Europäisches Patentamt

European Patent Office

Office européen des brevets

(11) Veröffentlichungsnummer: **0 442 138 B1**

# EUROPÄISCHE PATENTSCHRIFT

(45) Veröffentlichungstag der Patentschrift: **27.07.94**

(21) Anmeldenummer: **90125364.1**

(22) Anmeldetag: **22.12.90**

(51) Int. Cl.⁵: **G01N 31/00**, G01N 33/00, G01N 25/14

---

(54) Verfahren und Vorrichtung zur kontinuierlichen Überwachung von Abgasen aus Verbrennungsanlagen.

---

(30) Priorität: **24.01.90 DE 4001979**

(43) Veröffentlichungstag der Anmeldung:
**21.08.91 Patentblatt 91/34**

(45) Bekanntmachung des Hinweises auf die
Patenterteilung:
**27.07.94 Patentblatt 94/30**

(84) Benannte Vertragsstaaten:
**AT CH FR GB LI SE**

(56) Entgegenhaltungen:
**EP-A- 0 122 248**
**EP-A- 0 172 521**
**DE-A- 3 704 533**
**DE-A- 3 803 173**

(73) Patentinhaber: **KERNFORSCHUNGSZENTRUM
KARLSRUHE GMBH
Postfach 36 40
D-76050 Karlsruhe(DE)**

Patentinhaber: **DR. SEITNER MESS- UND RE-
GELTECHNIK GmbH
Mühlbachstrasse 20
D-82229 Seefeld(DE)**

(72) Erfinder: **Braun, Hartmut, Dr.
Lebrechtstrasse 45d
W-7500 Karlsruhe(DE)**
Erfinder: **Gerig, Andreas
Brahmsstrasse 1
W-7500 Karlsruhe(DE)**

(74) Vertreter: **Gottlob, Peter
Kernforschungszentrum Karlsruhe GmbH
Stabs. Patente und Lizenzen
Weberstrasse 5
D-76133 Karlsruhe (DE)**

---

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

**Beschreibung**

Die Erfindung betrifft ein Verfahren zur kontinuierlichen Überwachung von Abgasen aus Verbrennungsanlagen, die flüchtige Quecksilberhalogenide und ggf. metallisches Quecksilber in Dampfform enthalten, bezüglich ihres gesamten Quecksilbergehalts entsprechend dem Oberbegriff des ersten Patentanspruches sowie eine Vorrichtung zur Durchführung dieses Verfahrens.

Ein solches Verfahren ist aus der DE-OS 37 04 533 bekannt.

Bei diesem Verfahren wird dem Abgas ein kontinuierlicher Probengasstrom entnommen und zur Reduktion der flüchtigen Quecksilberhalogenide bei einer Temperatur von ca. 350° C durch ein Aktivkohlebett geleitet.

Hierbei werden Quecksilberhalogenide, insbesondere $HgCl_2$, an der Aktivkohle zu metallischem Quecksilber reduziert. Der gesamte Quecksilbergehalt im Abgas wird durch Messung des Gehaltes an metallischem Quecksilber im vorbehandelten Probenstrom ermittelt.

Nachteilig bei diesem Verfahren ist, daß sich im Aktivkohlebett störende Stoffe ansammeln können, die die Reduktion des Quecksilbers oder seine Freisetzung aus dem Aktivkohlebett behindern oder bereits reduziertes Quecksilber wieder oxidieren.

Aus diesem Grund ist das Verfahren nur im sogenannten Reingas, insbesondere bei Chlorwasserstoff-Konzentrationen unterhalb 100 $mg/m^3$, anwendbar. Ferner muß die Aktivkohle von Zeit zu Zeit ausgestauscht werden.

Weiterhin ist bekannt, den Probengasstrom anstelle durch ein Aktivkohlebett durch eine Zinn(II)-chlorid- oder durch eine Borhydridlösung zu leiten, wobei ebenfalls Quecksilberhalogenide zu metallischem Quecksilber reduziert werden.

Ein solches Verfahren bedingt eine kontinuierliche Zudosierung von Reduktionsmitteln. Der Gehalt an Reduktionsmittel in der Lösung muß laufend überwacht werden.

Der eingangs genannten Offenlegungsschrift kann ferner ein Alternativverfahren entnommen werden, bei dem auf Reduktionsmittel aller Art verzichtet wird und bei dem die Reduktion der Quecksilberhalogenide bei einer Temperatur von über 700° C erfolgt. Dabei muß vorhandener Chlorwasserstoff durch Calciumverbindungen abgebunden werden. Die hohe Verfahrenstemperatur macht das Verfahren verfahrenstechnisch aufwendig.

Aufgabe der Erfindung ist, ein Verfahren zur kontinuierlichen Überwachung der gesamten Quecksilberemission aus Abgasen von Verbrennungsanlagen, vor allem von Müllverbrennungsanlagen, vorzuschlagen, das nicht die Nachteile der bekannten Verfahren aufweist. Das Verfahren soll ohne betriebsfremde Reduktionsmittel durchführbar sein und keine hohen Temperaturen benötigen. Quecksilberhalogenide, insbesondere die flüchtigen Quecksilberchloride und -bromide, sollen zuverlässig zu Hg(O) reduziert werden.

Das Verfahren soll von der Zusammensetzung der Verbrennungsabgase weitgehend unabhängig sein. Weiterhin sollen sich die in der TA-Luft vorgeschriebenen Grenzwerte von 100 $\mu g/m^3$ für Quecksilberverbindungen zuverlässig überwachen lassen.

Ferner soll eine Vorrichtung zur Durchführung des Verfahrens vorgeschlagen werden.

Die Aufgabe wird erfindungsgemäß durch ein Verfahren mit den im Kennzeichen des ersten Patentanspruches genannten Merkmalen gelöst.

Der Anspruch 4 gibt eine Vorrichtung zur Durchführung des Verfahrens an.

Das erfindungsgemäße Verfahren zeichnet sich dadurch aus, daß die Reduktion von Quecksilberhalogeniden im Probengasstrom allein durch die vorhandenen reduzierenden Komponenten erfolgt. Bisherige Untersuchungen deuten darauf hin, daß die im Abgas vorhandenen Staubpartikel und der $SO_2$-Gehalt in der Lage sind, in flüssiger Phase Quecksilberhalogenide zu metallischem Quecksilber zu reduzieren.

Erfindungsgemäß wird ein Teilstrom des Abgasstroms (der Probengasstrom) soweit abgekühlt, daß sich ein Kondensat bildet. Prinzipiell ist es ausreichend, die Temperatur des Probengasstromes nur soweit zu verringern, daß eine Kondensation erfolgt. Vorteilhafter ist es jedoch, eine Temperatur im Bereich von 1 bis 30° C zu wählen. Innerhalb dieses Bereichs werden mit niedrigen Temperaturen, etwa zwischen 1 und 15° C, die besten Ergebnisse erzielt.

Mit dem gebildeten Kondensat wird der gasförmige Probengasstrom kontinuierlich in Kontakt gehalten. Dabei kühlt sich der Probengasstrom soweit ab, daß laufend frisches Kondensat erzeugt wird.

Die Inhaltsstoffe des Kondensats sind in der Lage, vorhandene Quecksilberhalogenide zu metallischem Quecksilber zu reduzieren. Durch die Tatsache, daß laufend frisches Kondensat nachgebildet wird, bleibt kontinuierlich eine ausreichende reduzierende Wirkung bestehen.

Im allgemeinen wird das Kondensat einen pH-Wert im schwach sauren Bereich aufweisen. Wenn das Kondensat sehr sauer ist, wie dies z. B. bei Müllverbrennungsanlagen, in denen stark chlorhaltige Stoffe verbrannt werden, der Fall ist, sollte der pH-Wert auf einen Bereich zwischen 3 und 8 eingestellt werden.

Besonders bevorzugt wird ein pH-Wert von etwa 3.

Wie erwähnt, werden im Probengasstrom vorhandene Quecksilberhalogenide in Kontakt mit dem Kondensat des Probenstroms zu metallischem Quecksilber reduziert. Das gebildete Quecksilber wird durch die Gasströmung aus der flüssigen Phase entfernt. Nur bei einem sehr geringen Gasfluß und wenn größere Volumenbereiche des Kondensats vom Gasfluß nicht erfaßt werden, kann es notwendig sein, das gebildete Quecksilber durch ein zusätzliches Trägergas aus der flüssigen Phase auszutreiben.

Zum Nachweis des metallischen Quecksilbers können die bekannten Analysenmethoden verwendet werden. Sehr gut eignet sich die in der eingangs genannten DE-OS 37 04 533 angesprochene Atomabsorptionsspektroskopie. Weiterhin kann z. B. die Massenspektroskopie verwendet werden, die insbesondere dann vorteilhaft ist, wenn noch andere Abgaskomponenten überwacht werden sollen.

Zur Durchführung des Verfahrens eignet sich eine Vorrichtung, die einen gekühlten, mit Kondensat befüllbaren Raum, eine Einrichtung zum Konstanthalten des Füllstands und eine Zuführung für den Probenstrom, die in der flüssigen Kondensat-Phase endet, aufweist. Der gekühlte Raum für das Kondensat kann entweder von einem Kühlmantel umschlossen sein oder im Innern Kühlschlangen oder Kühlfinger aufweisen.

Am einfachsten wird das Kondensat-Niveau durch einen Überlauf konstant gehalten. Vorzugsweise wird der Überlauf in der Weise angebracht, daß die flüssige Kondensatphase durch den Überlauf zusätzlich in Bewegung gehalten wird. Der Ort, an dem der Probenstrom kondensiert, soll von dem Ort, an dem das Kondensat durch den Überlauf abfließt, eine gewisse Strecke entfernt sein. Hierdurch wird erreicht, daß für die Reduktion der Quecksilberhalogenide immer frisches Kondensat zur Verfügung steht. Der Füllstand des Kondensats kann aber auch durch die üblichen anderen Methoden, z. B. durch eine Pumpe, die durch einen Füllstandsschalter gesteuert ist, konstant gehalten werden. Das Ende der Zuführung für den Probenstrom endet vorzugsweise unterhalb des konstant gehaltenen Kondensat-Füllstands und ist als Gasfritte ausgebildet.

Der nicht kondensierbare Anteil des Probenstroms und das metallische Quecksilber verlassen den gekühlten Raum durch eine in dessen oberem Bereich angebrachte Leitung, die gegen Ansaugen von flüssiger Phase ausreichend geschützt ist.

Insbesondere bei tiefen Kondensat-Temperaturen empfiehlt es sich, diese Leitung zu heizen. Durch die Leitung wird das metallische, gasförmige Quecksilber dem Quecksilberanalysator zugeführt.

Das erfindungsgemäße Verfahren eignet sich für die Abgasüberwachung von Verbrennungsanlagen, bei denen mit der Emission von Quecksilberhalogeniden gerechnet werden muß und insbesondere für Müllverbrennungsanlagen. Dabei ist es bedeutungslos, ob neben Quecksilberhalogeniden noch metallisches, gasförmiges Quecksilber im Abgasstrom vorhanden ist. Wie eigene Untersuchungen gezeigt haben, wird Quecksilber aus Verbrennungsanlagen meist als metallisches Quecksilber und/oder als Quecksilberhalogenid emittiert. Durch das erfindungsgemäße Verfahren werden Quecksilberhalogenide zuverlässig zu metallischem Quecksilber reduziert, so daß anschließend durch Bestimmung der Quecksilbermenge die gesamte Quecksilberemission unabhängig von der chemischen Form erfaßt werden kann.

Die Erfindung wird im folgenden anhand von Figuren und eines Durchführungsbeispiels näher erläutert.

Die Figuren 1 und 2 zeigen Vorrichtungen zur Durchführung des erfindungsgemäßen Verfahrens.

In Figur 3 ist der zeitliche Verlauf der Quecksilberemission an der Müllverbrennungsanlage TAMARA des Kernforschungszentrums Karlsruhe dargestellt.

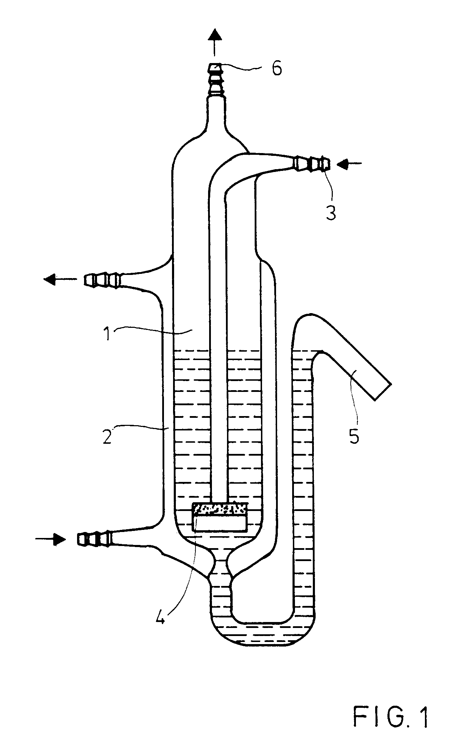

Die Vorrichtung nach Fig. 1 kann verwendet werden, wenn der Druck im Abgas- bzw. Probenstrom kleiner oder gleich dem Atmosphärendruck ist.

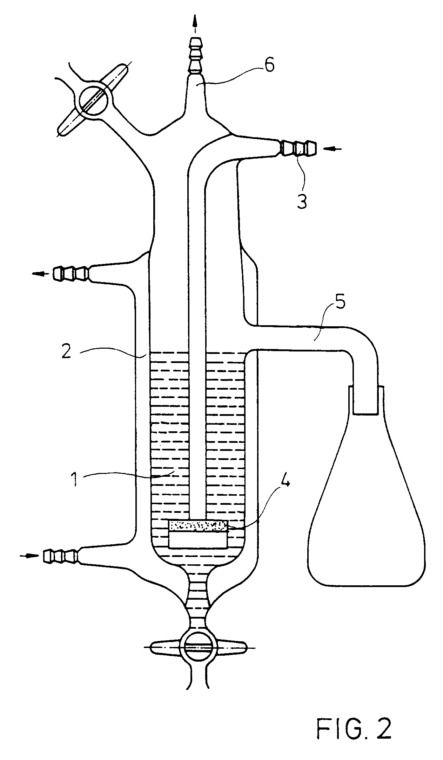

Die Vorrichtung nach Fig. 2 kann verwendet werden, wenn der Druck im Abgas- bzw. Probenstrom größer als der Atmosphärendruck ist.

Die Vorrichtungen nach Fig. 1 und 2 weisen einen von einem Kühlmantel 2 umschlossenen, gekühlten Raum 1 auf, der das Kondensat aufnimmt. Die Zuführung für den Probengasstrom 3 endet in einer Gasfritte 4 unterhalb des durch einen Kondensat-Überlauf 5 konstant gehaltenen Kondensatfüllstands.

Der Probengasstrom gelangt durch den Ausgang 6 in den Quecksilberanalysator. Der Probengasstrom wird mit Hilfe einer Pumpe (nicht dargestellt) in den Raum 1 gefördert.

Das erfindungsgemäße Verfahren wird im folgenden Beispiel unter Verwendung der Vorrichtung nach Fig. 1 mit den Ergebnissen einer diskontinuierlichen Probenahme verglichen.

Die Probenahme erfolgte am Kamin der Müllverbrennungsanlage TAMARA des Kernforschungszentrums Karlsruhe, die mit einem Durchsatz von 200 kg Hausmüll/Stunde betrieben wurde. Über eine beheizte Sonde wurde dem Reingas ein Probegasstrom von 120 l/h entnommen und mit Hilfe einer Pumpe der Vorrichtung nach Fig. 1 zugeführt.

Der Probegasstrom wurde auf eine Temperatur von 10° C abgekühlt.

Die Bildungsrate an Kondensat betrug bei dieser Temperatur etwa 0,015 l/h; die Menge an Kondensat im gekühlten Kondensatraum wurde bei 0,1 l gehalten.

Der pH-Wert des Kondensat lag während des gesamten Versuchs bei 3.

Die nicht kondensierten Anteile des Probengasstroms, die das ursprünglich vorhandene und das neu gebildete metallische Quecksilber enthielten, wurden bei Atmosphärendruck in eine Küvette geleitet. Mit Hilfe der Atomabsorptionsspektroskopie wurde bei der Wellenlänge 253,7 nm kontinuierlich der Quecksilbergehalt gemessen.

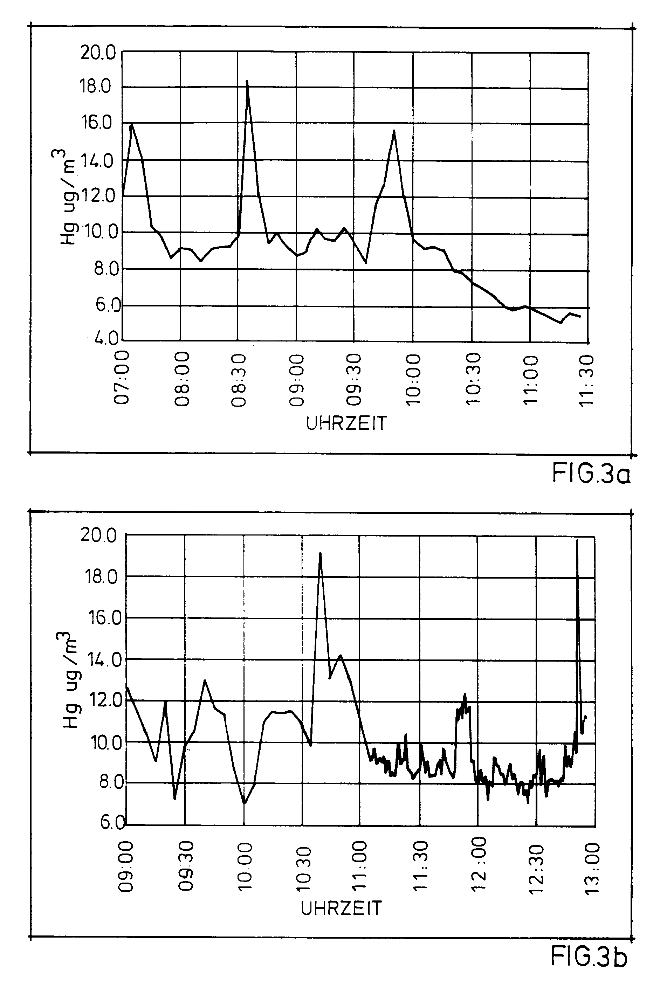

Fig. 3 veranschaulicht den zeitlichen Verlauf der Quecksilberemission.

Die Meßwerte wurden mit Hilfe eines Rechners gespeichert und über eine Zeit von vier Stunden aufsummiert.

In der Tabelle sind diese aufsummierten Meßwerte aufgeführt.

Zur gleichen Zeit wurde am Kamin ein zweiter, gleich großer Probengasstrom mit einer zweiten beheizbaren Sonde entnommen.

Dieser Probengasstrom wurde jeweils vier Stunden lang über eine Kombination der Feststoffabsorber Dowex 1x8 und Jodkohle geleitet. Der Anionenaustauscher eignet sich in hervorragender Weise zur selektiven $HgCl_2$-Abscheidung, während Jodkohle ein Sorptionsmittel für Gesamtquecksilber ist, bei Kombination mit Dowex entsprechend für Hg(O). Die beaufschlagten Feststoffadsorber wurden anschließend chemisch auf Quecksilber analysiert. Die Analysenergebnisse sind ebenfalls in der Tabelle dargestellt.

Die nach beiden Meßverfahren erhaltenen Ergebnisse stimmen gut miteinander überein.

## Tabelle

| Versuch Nr. | Erfaßtes Rauchgasvolumen in l | erfindungsgemäßes Verfahren kontinuierlich, Summation über 4 Stunden) in $\mu g/m^3$ als Hg(O) | Vergleichsverfahren diskontinuierlich 4-Stunden-Werte | in $\mu g/m^3$ |
| --- | --- | --- | --- | --- |
| | | | Hg-Halogenide | Hg(O) |
| 1 | 428 | 8 | 8 | 1 |
| 2 | 376 | 10 | 8 | <1 |
| 3 | 454 | 9 | 7 | 1 |
| 4 | 441 | 9 | 8 | 1 |

**Patentansprüche**

1. Verfahren zur kontinuierlichen Überwachung von Abgasen aus Verbrennungsanlagen, die flüchtige Quecksilberhalogenide und ggf. metallisches Quecksilber in Dampfform enthalten, bezüglich ihres gesamten Quecksilbergehalts, bei dem
   a) den Gasen ein kontinuierlicher Probengasstrom entnommen wird, danach
   b) die im Probengasstrom enthaltenen Quecksilberhalogenide durch Reduktionsmittel zu metallischem Quecksilber reduziert werden und anschließend
   c) der gesamte Gehalt an metallischem Quecksilber durch eine geeignete Analysenmethode ermittelt wird,
   dadurch gekennzeichnet, daß
   d) der Probengasstrom soweit abgekühlt wird, daß sich ein Kondensat bildet und
   e) der Probengasstrom kontinuierlich mit dem Kondensat in Kontakt gehalten wird.

**2.** Verfahren nach Anspruch 1, dadurch gekennzeichnet, daß der Probengasstrom auf eine Temperatur zwischen 1 und 30° C abgekühlt wird.

**3.** Verfahren nach Anspruch 1, dadurch gekennzeichnet, daß das Kondensat auf einem pH-Wert von mindestens 3 und höchstens 8 gehalten wird.

**4.** Vorrichtung zur Durchführung des Verfahrens nach Anspruch 1, gekennzeichnet durch
f) einen kühlbaren, mit Kondensat befüllbaren Raum,
g) eine Einrichtung, mit deren Hilfe sich der Füllstand des Kondensats konstant halten läßt,
h) eine Zuführung für den Probengasstrom, die im gekühlten Raum endet.
i) eine Gasleitung, die den Raum oberhalb des Kondensat-Füllstandes mit einem Quecksilberanalysator verbindet.

**5.** Vorrichtung nach Anspruch 4, dadurch gekennzeichnet, daß die Einrichtung zum Konstanthalten des Füllstandes ein Überlauf ist.

**6.** Vorrichtung nach Anspruch 4, dadurch gekennzeichnet, daß das Ende der Zuführung für den Probengasstrom als Gasfritte ausgebildet ist und sich unterhalb des konstant gehaltenen Füllstandes befindet.

## Claims

**1.** Method of continuously monitoring waste gases from combustion systems, which waste gases contain volatile mercury halides and possibly metallic mercury in vapour form, in respect of their entire mercury content, wherein
a) a continuous sample gas stream is removed from the gases, then
b) the mercury halides contained in the sample gas stream are reduced by reducing agents to metallic mercury, and subsequently
c) the entire content of metallic mercury is determined by a suitable analysing process, characterised in that
d) the sample gas stream is cooled to such an extent that a condensate is formed, and
e) the sample gas stream is continuously kept in contact with the condensate.

**2.** Method according to claim 1, characterised in that the sample gas stream is cooled to a temperature between 1 and 30° C.

**3.** Method according to claim 1, characterised in that the condensate is kept at a pH value of at least 3 and at most 8.

**4.** Apparatus for accomplishing the method according to claim 1, characterised by
f) a coolable chamber which can be filled with condensate,
g) a means, whereby the level of the condensate can be kept constant,
h) a supply line for the sample gas stream, which line terminates in the cooled chamber,
i) a gas pipe which connects the space above the condensate level to a mercury analyser.

**5.** Apparatus according to claim 4, characterised in that the means for keeping the level constant is an overflow.

**6.** Apparatus according to claim 4, characterised in that the end of the supply line for the sample gas stream is a gas frit and is situated beneath the level which is kept constant.

## Revendications

**1.** Procédé de surveillance en continu de gaz de fumées d'unités d'incinération, qui contiennent des halogénures de mercure volatils et, le cas échéant, du mercure métallique sous forme de vapeur, du point de vue de leur teneur totale en mercure, dans lequel :
a) on prélève dans les gaz un courant de gaz échantillon en continu, ensuite,
b) on réduit les halogénures de mercure contenus dans le courant de gaz échantillon par un réducteur de mercure métallique et ensuite,

c) on détermine la teneur totale en mercure métallique par un procédé d'analyse approprié,, caractérisé en ce que :

d) on refroidit le courant de gaz échantillon de façon à former un condensat et

e) on maintient en continu le courant de gaz échantillon en contact avec le condensat.

2. Procédé selon la revendication 1, caractérisé en ce qu'on refroidit le courant de gaz échantillon à une température comprise entre 1 et 30°C.

3. Procédé selon la revendication 1, caractérisé en ce qu'on maintient le condensat à un pH d'au moins 3 et au plus de 8.

4. Dispositif pour réaliser le procédé selon la revendication 1, caractérisé par :

f) une chambre réfrigérante remplissable de condensat,

g) un dispositif permettant de maintenir constant le niveau du condensat,

h) une introduction du courant de gaz échantillon qui débouche dans la chambre refroidie,

i) une conduite de gaz qui relie la chambre au-dessus du niveau du condensat à un analyseur de mercure.

5. Dispositif selon la revendication 4, caractérisé en ce que le dispositif pour maintenir constant le niveau est un trop plein.

6. Dispositif selon la revendication 4, caractérisé en ce que l'extrémité d'introduction du courant de gaz échantillon est en forme de fritte pour du gaz et se trouve maintenu constamment en dessous du niveau.

FIG. 1

FIG. 2

FIG.3a

FIG.3b